# EUROPEAN PATENT APPLICATION

(11) **EP 4 183 874 A1**
(43) Date of publication of application: **24.05.2023**
(21) Application number: 21841351.6
(22) Date of filing: 02.06.2021
(51) Int. Cl.: C12N 5/10, A61K 39/00, A61P 35/00, C12Q 1/02

(54) **TCR-T CELL FOR KILLING TUMORS, AND PREPARATION METHOD THEREFOR AND USE THEREOF**

(30) Priority: 14.07.2020 CN 202010674259
(71) Applicant: Guangzhou Finelmmune Biotechnology Co., Ltd., Guangzhou, Guangdong 510530 (CN)
(72) Inventor: LIU, Haiping, Guangzhou, Guangdong 510530 (CN); LI, Xiang, Guangzhou, Guangdong 510530 (CN); LI, You, Guangzhou, Guangdong 510530 (CN); BIAN, Chaochao, Guangzhou, Guangdong 510530 (CN)
(74) Representative: Appleyard Lees IP LLP
(86) International application number: PCT/CN2021/097813
(87) International publication number: WO 2022/012198

(57) **Abstract**

The present disclosure provides a TCR-T cell for tumor-killing, wherein the TCR-T cell is a T cell carrying a TCR that recognizes a tumor antigen, and the TCR in the TCR-T cell is derived from any one or more of the following T cells: 1) a CD4 T cell expressing one or more of TNFRSF18, CXCL13, TNFRSF4, TNFSF8, ENTPD1, ACP5, LAYN, TNFRSF9, CTLA4, CD200 and TIGIT genes in the tumor; and 2) a CD8 T cell expressing one or more of TNFRSF18, CXCL13, CXCR6, GALNT2, ENTPD1, ACP5, HAVCR2, LAYN, TNFRSF9, CTLA4 and CD109 genes in the tumor. The TCR-T cell according to the present disclosure can be effectively applied to the treatment of a tumor, especially in an immunotherapy.

## Description

### TECHNICAL FIELD

The present disclosure relates to the technical field of immunotherapy, in particular to a TCR-T cell for tumor-killing, a preparation method and use thereof.

### BACKGROUND

T cells recognize antigens presented by histocompatibility molecules (Human Leukocyte Antigen, HLA) on the surface of target cells via T Cell Receptors (TCR) on the surface thereof, thus achieving direct attack and killing of the target cells. Traditional TCR-T cell therapy for recognizing tumor antigens are directed at known tumor antigens and involve cloning TCRs that can recognize these known antigens and determining the HLA subtypes corresponding thereto. A patient who expresses both the tumor antigens and HLA subtypes is then selected to prepare TCR-T cells for re-infusion therapy. However, currently known tumor antigens are few, and these antigens are only expressed in a small number of patients. Moreover, it is required to express the corresponding HLA molecules simultaneously, which leads to very few patients who can meet the both requirements. Most patients cannot be treated by such TCR-T cells that recognize the known tumor antigens.

In tumor cells, amino acid sequence variants caused by gene mutations are the main source of tumor antigens. However, since mutations in tumors are random, and these tumor antigens produced by random mutations are usually unique to each patient. That is to say, there are very few tumor antigens shared among the patients. Therefore, in order to achieve effective treatment for most patients, it is necessary to establish corresponding TCR-T cells that recognize the tumor antigens specific to each patient.

During the occurrence and development of tumors, T cells that recognize tumor antigens in the body will be activated by the tumor antigens and enter tumor tissues to expand and kill tumor cells in the tumors. Therefore, if it is possible to identify and isolate T cells that recognize tumor antigens from the tumors in patients, obtain the TCR sequences carried thereby and enable expression thereof in the peripheral blood T cells of the patients, a TCR-T cell for tumor antigen recognizing derived from the patients themselves, i.e. an individualized TCR-T cell, will be established for individualized tumor therapy.

However, tumors are interconnected with the lymphatic and blood systems between normal tissues and blood, and a large number of T cells that do not recognize tumor antigens enter the tumors through the circulation of the blood and lymphatic systems. Therefore, in addition to the T cells that recognize the tumor antigens, the tumor tissue also contains a large number of T cells that do not recognize tumor antigens from the adjacent tissues and blood, that is, there is a mixture of various T cells. To obtain the TCRs that can recognize tumor antigens from such mixed T cells, it is necessary to first determine, by tumor antigen stimulation, which T cells can recognize the tumor antigens and then obtain the TCR sequences carried by these T cells for individualized TCR-T cell therapy. In addition, after whether these T cells recognize tumor antigens is ascertained, the characteristics of gene expression of these T cells are analyzed, the differences of the molecular characteristics between the T cells that recognize the tumor antigens and that do not recognize are compared and identified to find out the molecular markers specific to the T cells that can recognize the tumor antigens, and by means of these specific molecular markers, T cells recognizing the tumor antigens in the tumors can be isolated and obtained for subsequent treatment.

Identifying whether a T cell recognizes a tumor antigen is a systematic project with complicated process and high technical requirements. First of all, it is necessary to isolate T cells from a tumor tissue and culture the T cells *in vitro.* In addition, it is also necessary to identify the tumor antigen in the tumor tissue and then stimulate the isolated T cells *in vitro* with the identified tumor antigen. Only tumor antigen stimulation is the direct approach to prove if a T cell can recognize the tumor antigen. Since the whole experimental process of identification is extremely complicated, along with the high technical difficulty, systematic identification and analysis of tumor antigens in T cells in a tumor tissue currently remains impossible. As a result, it is currently also impossible to quickly obtain T cells and TCRs that recognize tumor antigens from a tumor tissue for treatment.

### SUMMARY

In view of the above technical problems to be solved, an object of the present disclosure is to provide a technical solution that provides a recognition capacity to accurately and quickly analyze tumor antigens of a T cell, in which by comparing the gene expression between T cells that recognize a tumor antigen and that do not recognize, the molecular markers specific to the T cells that recognize the tumor antigen in a tumor tissue are found out, and by means of these molecular markers, quick separation of the tumor antigen-recognizing T cells can be achieved, thus quickly obtaining TCRs that recognizes the tumor antigens, so as to establish an individualized therapeutic technique with the TCR-T cells recognizing the tumor antigens.

In order to achieve the above object, the present disclosure provides a TCR-T cell for tumor-killing, wherein the TCR-T cell is a T cell carrying a TCR that recognizes a tumor antigen, and the TCR in the TCR-T cell is derived from any one or more of the following T cells:
1) a CD4 T cell expressing one or more of TNFRSF18, CXCL13, TNFRSF4, TNFSF8, ENTPD1, ACP5, LAYN, TNFRSF9, CTLA4, CD200 and TIGIT genes in the tumor; and
2) a CD8 T cell expressing one or more of TNFRSF18, CXCL13, CXCR6, GALNT2, ENTPD1, ACP5, HAVCR2, LAYN, TNFRSF9, CTLA4 and CD109 genes in the tumor.

The present disclosure further provides use of the TCR-T cell in the preparation of a medicine for treating a tumor.

Preferably, the tumor comprises but not limited to any one or more selected from the group consisting of lung cancer, melanoma, intestinal cancer, liver cancer, stomach cancer, breast cancer, cervical cancer, ovarian cancer, kidney cancer, bladder cancer and esophageal cancer.

The present disclosure further provides a method for preparing the TCR-T cell, comprising: introducing one or more nucleotide sequences of a TCR that recognizes a tumor antigen into a T cell for expression to construct the TCR-T cell.

Preferably, the step of identifying the TCR that recognizes the tumor antigen comprises: by means of flow sorting, magnetic bead separation or tumor tissue in-situ sequencing, obtaining, from a tumor tissue, a T cell, which expresses any one or more of the markers of a CD4 T cell for tumor antigen recognition, selected from the group consisting of TNFRSF18, CXCL13, TNFRSF4, TNFSF8, ENTPD1, ACP5, LAYN, TNFRSF9, CTLA4, CD200 and TIGIT, and a TCR sequence carried thereby; and a T cell, which expresses any one or more of the markers of a CD8 T cell for tumor antigen recognition, selected from the group consisting of TNFRSF18, CXCL13, CXCR6, GALNT2, ENTPD1, ACP5, HAVCR2, LAYN, TNFRSF9, CTLA4 and CD109, and a TCR sequence carried thereby.

The present disclosure further provides a method for identifying a T cell and a TCR for tumor antigen recognition, comprising: establishing a TCR-expressing TCR-T cell by cloning a high-frequency TCR in a tumor tissue, performing *in vitro* tumor antigen stimulation by using antigen-presenting cells expressing a tumor antigen tandem gene, and identifying a TCR carried by a TCR-T cell that can be stimulated as the TCR for tumor antigen recognition, and the T cell carrying such TCR as the T cell for tumor antigen recognition,
wherein the TCR in the TCR-T cell is derived from any one or more of the following T cells:
1) a CD4 T cell expressing one or more of TNFRSF18, CXCL13, TNFRSF4, TNFSF8, ENTPD1, ACP5, LAYN, TNFRSF9, CTLA4, CD200 and TIGIT genes in the tumor; and
2) a CD8 T cell expressing one or more of TNFRSF18, CXCL13, CXCR6, GALNT2, ENTPD1, ACP5, HAVCR2, LAYN, TNFRSF9, CTLA4 and CD109 genes in the tumor.

The TCR-T cell according to the present disclosure can be effectively applied to the treatment of a tumor, especially in an immunotherapy.

### BRIEF DESCRIPTION OF DRAWINGS

Fig. 1 shows the stimulation responses of high-frequency TCRs in CD4 T cells and CD8 T cells to tumor antigens of tumors.
Fig. 2 shows that TCR-T cells constructed with TCRs from a subset of tumor antigen-recognizing T cells can effectively treat the tumor (* P < 0.05; ** P < 0.01).
Fig. 3 shows the molecular marker genes of a subset of tumor antigen-recognizing T cells, wherein (a) shows the difference of the specific gene expression in CD4 T cells and (b) shows the difference of the specific gene expression in CD8 T cells.
Fig. 4 shows that T cells sorted by molecular markers of tumor antigen-recognizing T cells can effectively recognize tumor antigens (* P < 0.05; ** P < 0.01), wherein (a) represents CD4 T cells and (b) represents CD8 T cells.

### DETAILED DESCRIPTION

In order to better explain the object, technical solution and advantages of the present disclosure, the present disclosure will be further explained hereinafter with reference to exemplary implementations. It should be noted that for the sake of brevity and clarity, some conventional technical operation steps, reagents and instruments may not be described in detail in the following exemplary implementations, and it should be understood that these conventional technical operation steps, reagents and instruments are obvious to those of ordinary skill in the art unless otherwise specified.

The present disclosure will be further described hereinafter with reference to exemplary implementations, and the advantages and features of the present disclosure may become apparent upon reading the description. However, the implementations are merely illustrative and are not intended to limit the scope of the present disclosure. Those skilled in the art should understand that the details and forms of the technical solution according to the present disclosure can be modified or replaced without departing from the spirit and scope of the present disclosure, and these modifications and replacements are all within the scope of protection of the present disclosure.

Firstly, DNA was extracted from a tumor tissue using DNeasy Blood & Tissue Kit (Art. No. 69506) from Qiagen, and RNA was extracted from the tumor tissue using miRNeasy-Mini Kit (Art. No. 217004) from Qiagen. The obtained DNA and RNA samples were then sent to a sequencing service company for exon and RNA sequencing. Moreover, DNA extracted from peripheral blood leukocytes was sent to the sequencing service company for exon sequencing, as a reference gene sequence for detecting gene mutation in the tumor. By comparing the exon sequences of the tumor tissue and the peripheral blood leukocytes, the mutation sites and the corresponding genes in the tumor tissue were found. In addition, according to the amount of expression of the RNA of each mutant gene in the tumor tissue, the expression abundances of the mutant genes in the tumor were ranked, and the mutants with high expression in the tumor tissue were selected as tumor antigens. Then, a 25 amino acid epitope peptide centered on the mutation site and a new polypeptide produced by frameshift mutation were connected in series as a tandem tumor antigen, a gene of the connected tumor mutant antigen in series was synthesized and expressed in immortalized B cells from a patient infected by EBV virus, whereby antigen-presenting cells covering most of the tumor mutant antigens were established and could be used for tumor antigen stimulation *in vitro,* thereby solving the difficult problem of failing to effectively obtain tumor antigens for antigen presentation in traditional methods.

Secondly, T cells in the tumor were sorted by flow cell sorting technology, and then, each of the T cells were subjected to RNA sequencing. Firstly, the tumor tissue was cut into pieces smaller than 1 mm³ with a scalpel, and the pieces were then added to a 10-fold volume of tissue digestion solution (150 ml of RIPA 1640 medium, 3 ml of fetal bovine serum, 27 mg of collagenase, and 7.5 mg of DNAse) and digested at 37°C for 2 h. After complete digestion, the digested solution was filtered through a 70 µM cell strainer, washed and resuspended with PBS to obtain a single-cell suspension. A fluorescent flow antibody (color scheme: CD45-APC-Cy7, CD3-APC, and CD19-PE) was added to the sample with the final concentration of 1 :200; and after staining at 4°C for 30 min, the sample was washed twice with PBS, and individual T lymphocytes (CD45+ CD3+) were sorted into a 96-well plate by a flow cell sorter (model: BD Bioscience Arial III). Then, single-cell RNA reverse transcription and cDNA amplification were carried out in the 96-well plate, and a library was constructed and sent to the sequencing service company for sequencing. The sequencing platform was Illumina HiSeq X Ten. The sequencing results were analyzed by CellRanger software to obtain the levels of the gene expression in each of the T cells and the TCR sequences carried thereby.

Then, the gene sequences of each TCR were synthesized and constructed into a lentiviral expression vector. Then, 293T cells were used to package the virus, a supernatant of the virus was collected, and the virus was concentrated by high-speed centrifugation. After the virus was resuspended, it was mixed with peripheral blood lymphocytes activated by CD3/CD28 antibody for 3 days. Polybrene with a final concentration of 8 µg/ml was added, and the mixture was then added to a 24-well plate and centrifuged at 30°C at 2000 rpm for 50 min for T cell infection. Then, the supernatant was removed, and RPMI medium containing 10% FBS and 200 ng/ml IL2 was added for culture. A TCR-T cell line was established for each TCR by lentivirus centrifugal infection method, so as to use *in vitro* for long term and solve the difficult problem of tumor T cell culture *in vitro* in traditional identification methods.

After the establishment of the above two technical methods, the identification and analysis of T cells for tumor antigen recognition were carried out on a plurality of tumor tissues of different tumor types, including tumor types such as lung cancer, melanoma, intestinal cancer, liver cancer, stomach cancer, breast cancer, cervical cancer, ovarian cancer, kidney cancer, bladder cancer and esophageal cancer.

The representative results from three lung cancer tissues are shown below.

Fresh tumor (lung cancer) tissues (Tumor-1, Tumor-2, and Tumor-3) were obtained, and divided into three portions. Among them, the first portions were used to construct a PDX (Patient-Derived Xenograft) tumor model in an immunodeficient NSG mouse. Firstly, the tumor tissues were cut into pieces of about 1 mm³ with sterile scissor and then subcutaneously inoculated into the left groin of the immunodeficient NSG mouse with trocar. After tumor was formed, it was cut into small pieces again and further inoculated into the NSG mouse. After tumor formation for three times, the tumor was cut into small pieces and frozen in liquid nitrogen for subsequent tumor treatment experiment. The second portions of tumor tissues were used to extract DNA and RNA, and the extracted DNA and RNA were then sent to the sequencing service company for exon and RNA sequencing. Moreover, the DNA extracted from peripheral blood leukocytes was sent to the sequencing service company for exon sequencing, as a reference gene sequence for detecting gene mutation in the tumor. The third portions of tumor tissues were dissociated into single cells in suspension according to the above experimental scheme, and single CD4 T cell and CD8 T cell were then sorted into 96-well plates by flow sorting. Then, these T cells were respectively subjected to RNA sequencing to obtain the TCR sequences of each cell and the RNA expression levels of most genes. According to the results of exon and RNA sequencing, the mutant genes in each tumor tissue and the expression levels of these mutant genes were obtained. Then, the genes expressing mutant sequences were genetically connected in an antigen tandem manner to obtain a synthesized gene, which was transduced into EBV-immortalized B cells obtained from a patient, whereby a B cell line presenting tumor mutant antigen was established.

The selected mutant antigens and the synthesized tandem antigen gene sequences are as shown in Tables 1 to 3. In addition, by analyzing the TCR sequences carried by each of the CD4 T cells and CD8 T cells, 10 TCRs with the highest frequencies, i.e. the most amplified TCRs, were selected separately from CD4 T cells and CD8 T cells of each tumor, and cloned and constructed into a lentivirus expression vector. The information of the selected TCRs is as shown in Table 4. The vector was then introduced into the corresponding peripheral blood T cells of patients via lentivirus to prepare TCR-T cells, and the TCR-T cell line was established for each TCR.

**Table 1: Tumor mutations of Tumor-1 and tandem antigen gene sequences**

| **Gene name** | **Reference sequence** | **Mutation type** | **Mutant base** | **Mutant amino acid** | **Tandem antigen sequence** |
|---|---|---|---|---|---|
| SGK1 | NM _001291995 | Missense mutation | G16A | E6K | MTVKTKAAKGTLTYSRMR |
| CPNE1 | NM_001198863 | Missense mutation | G505A | D169N | KSDPFLEFFRQGNGKWHLVYR SEVI |
| TMCC1 | NM_001017395 | Missense mutation | A278T | D93V | MDLESQNACAEIVGVPTHPTAL NRV |
| KRT7 | NM_005556 | Missense mutation | A553C | N185H | VEDFKNKYEDEIHHRTAAENEF VVL |
| CFH | NM_000186 | Missense mutation | G860A | G287E | GDYSPLRIKHRTEDEITYQCRN GFY |
| FGFR2 | NM_001144914 | Missense mutation | A1929T | E643D | VPSQRPTFKQLVDDLDRILTLT TNE |
| ITGB6 | NM_001282354 | Missense mutation | G1640A | R547Q | CIECHLSAAGQAQEECVDKCK LAGA |
| HIPK2 | NM_001113239 | Missense mutation | G1477C | E493Q | MTTDLEGSDMLVQKADRREFI DLLK |
| PAM | NM_000919 | Missense mutation | C790G | Q264E | NGQWTLIGRQSPELPQAFYPVG HPV |
| WWP2 | NM_199424 | Missense mutation | C388G | H130D | GGIAREWFFLLSDEVLNPMYCL FEY |
| GRB7 | NM_001030002 | Missense mutation | C946T | R316W | KPNKLRNGHKGLWIFCSEDEQ SRTC |
| PLXNA3 | NM_017514 | Missense mutation | G2689A | E897K | PAEYISAERIVCKMEESLVPSPP PG |
| RAVER2 | NM_018211 | Missense mutation | G626T | G209V | AKARLELLGRQLVASALFAQW MDVN |
| WDFY4 | NM_020945 | Missense mutation | C8010G | F2670L | LQGGSFDVADRMLHSVKSTWE SASR |
| ZMYM3 | NM_001171162 | Missense mutation | G980A | R327H | RTPLQKGQTAYQHKGLPQLFC SSSC |
| KIAA1109 | NM_015312 | Missense mutation | A3680C | H1227P | TQHLSLQVPLRSPSSSSSSEENS SS |
| NUP188 | NM_015354 | Missense mutation | G3416A | G1139E | VVRRQLFLDVLDETKALLLVP ASVN |
| PWP2 | NM_005049 | Missense mutation | A2644C | T882P | MQYALAVSKQRGPKRSLDPLG SEEE |
| RCHY1 | NM_001278537 | Missense mutation | A569C | K190T | GRSTVQFHILGMTCKICESYNT AQA |
| INSR | NM_001079817 | Missense mutation | G4030A | E1344K | DGGSSLGFKRSYKEHIPYTHMN GGK |
| PTPN14 | NM_005401 | Missense mutation | C2621T | S874L | KRPLMLAALNGLLVARVSGRE ENRV |
| SMYD2 | NM_020197 | Missense mutation | T983G | M328R | ELLEICELSQEKRSSVFEDSNVY ML |
| ZNF579 | NM_152600 | Missense mutation | G1588A | A530T | PQSPPAPPVFLSTSCFDSQDHSA FE |
| AKNA | NM_030767 | Missense mutation | G3436A | E1146K | KSTERLPGEPRGKEQIVPPGRQ RAR |
| ZC3H14 | NM_001160103 | Missense mutation | C9G | I3M | MEMGTEISRKIRSAI |
| RASGRF1 | NM_153815 | Missense mutation | C1342G | Q448E | 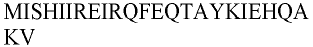 |
| TACC2 | NM_001291878 | Missense mutation | T363G | I121M | 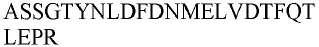 |
| PIAS3 | NM_006099 | Missense mutation | A562T | I188L | REVLPGAKCDYTLQVQLRFCL CETS |
| RETSAT | NM_017750 | Missense mutation | C1598T | A533V | LTNQFYLAAPRGVCYGADHDL GRLH |
| POLR3B | NM_001160708 | Missense mutation | C2633T | S878L | CPDIIMNPHGFPLRMTVGKLIEL LA |
| HGF | NM_000601 | Missense mutation | C1708A | L570M | DEKCKQVLNVSQMVYGPEGSD LVLM |
| RETSAT | NM_017750 | Missense mutation | G1606A | G536R | QFYLAAPRGACYRADHDLGRL HPCV |
| AIMP1 | NM_001142415 | Missense mutation | G654A | M218I | MVILLCNLKPAKIRGVLSQAM VMCA |
| ROBO1 | NM_001145845 | Missense mutation | C2908G | P970A | DSNLTTYSRPGQATPYATTQLI QSN |
| CUL7 | NM_001168370 | Missense mutation | C3208T | R1070C | WPVFREQLCRHTCLFYMVRAQ AWSQ |
| PAN2 | NM_001127460 | Missense mutation | G1801T | D601Y | EASALGLILADSYEASGKGNLA RLI |
| VPS13B | NM_017890 | Missense mutation | A5215T | S1739C | EVNITTNLDFFLCVAQVQLLHQ LIV |
| TMOD2 | NM_001142885 | Missense mutation | G55C | E19Q | KELEKYKNIDEDQLLGKLSEEE LKQ |
| UNC5C | NM_003728 | Deletion frame shift | 2551_2553d el | 851 851del - | GPSAFSIPLPIRQKLCSSLDAPQ TR |
| C8orf33 | NM_023080 | Deletion frame shift | 641_667del | 214 223del - | SQRVCRPRSIWRAKFRFNFF |
| DNAH10 | NM_207437 | Missense mutation | G3067A | E1023K | FAAKKPPCVAYDKKLQFYSKI AYEV |
| TMEM80 | NM_001042463 | Missense mutation | G242A | R81K | GGAGKMAAPRRGKGSSTVLSS VPLQ |
| SEC24C | NM_198597 | Missense mutation | G1403A | G468D | VPPQYFQHLDHTDKRVDAYDR PELS |
| ARMC9 | NM_001291656 | Missense mutation | A724C | I242L | RYNKIQADYHNLLGVTAELVD SLEA |
| ALMS1 | NM_015120 | Missense mutation | G9423C | L3141F | PSLPDSNTITQDFKTIPSQNSQIV T |
| PDZD2 | NM_178140 | Missense mutation | A1504C | K502Q | ESPKQGSNKIKLQSRLSGGVHR LES |
| IPO8 | NM_001190995 | Missense mutation | T393G | H131Q | QQAFNYLNQGVVQSITWKQM KPHIQ |
| TTN | NM_003319 | Missense mutation | A76952C | H25651P | ELSSLRYSSPQAPVKVEETRKD FRY |
| HDAC5 | NM_001015053 | Missense mutation | A101G | E34G | PRTSLHSIPVTVGVKPVLPRAM PSS |
| RAC3 | NM_001316307 | Missense mutation | G484C | D162H | GGLCEIPGVLSPHPAGPEDSV |
| ARMCX1 | NM_016608 | Missense mutation | G1138A | E380K | KVPSELISLFNKKWDREILLNIL TL |
| DNAH9 | NM_001372 | Missense mutation | G8743C | E2915Q | GEIPDLYSDDEVQNIISNVRNEV KS |
| FRG1 | NM_004477 | Missense mutation | A55G | T19A | VKSTKLVLKGTKAKSKKKKSK DKKR |
| UBAP1 | NM_001171201 | Missense mutation | C193G | R65G | TEPGRPAGASTFGLLRRRQQRH SGT |
| ANO1 | NM_018043 | Missense mutation | G340A | E114K | PGKGASLDAGSGKPPMDYHED DKRF |
| ZNF48 | NM_152652 | Missense mutation | G353A | G118D | ASSDRAAVCGECDKSFRQMSD LVKH |
| SRPK3 | NM_001170760 | Missense mutation | A625C | I209L | HGLDYLHTKCKILHTDIKPENIL LC |
| CDH11 | NM_001308392 | Missense mutation | T1937G | M646R | PSLMEPPSPREDRRLLYLGFQL MLF |
| KSR2 | NM_173598 | Missense mutation | G2666A | R889K | ILLFCWAFEQEEKPTFTKLMDM LEK |
| ADRA1B | NM_000679 | Insertion frame shift | 1122_1123in sCGCCGC | R374delinsRRR | LGCQCRGRGRRRRRRRRRRRRL GGCAY |

The nucleotide sequences of Tumor-1 tandem antigen genes are as shown in SEQ ID NO. 1 and SEQ ID NO. 2.

**Table 2: Tumor mutations of Tumor-2 and tandem antigen gene sequences**

| **Gene name** | **Reference sequence** | **Mutation type** | **Mutant base** | **Mutant amino acid** | **Tandem antigen sequence** |
|---|---|---|---|---|---|
| PCBP2 | NM_001098620 | Missense mutation | A220G | M74V | AGPTNAIFKAFAVIIDKLEEDIS SS |
| CTNNB1 | NM_001098209 | Missense mutation | T109C | S37P | WQQQSYLDSGIHPGATTTAPS LSGK |
| ACKR1 | NM_001122951 | Missense mutation | A893G | H298R | LLLNLAEALAILRCVATPLLL ALFC |
| PRRC2C | NM_015172 | Missense mutation | G6857C | S2286T | APPIATGVSSSATGPSTANYNS FSS |
| PAPSS1 | NM_005443 | Missense mutation | G964A | G322S | LTATHEDKERLDSCTAFALM YEGRR |
| HIST1H2AE | NM_021052 | Missense mutation | T197C | L66P | AVLEYLTAEILEPAGNAARDN KKTR |
| ATP5J | NM_001003696 | Missense mutation | T125C | L42P | VAFNKELDPIQKPFVDKIREY KSKR |
| KPNB1 | NM_001276453 | Missense mutation | G433A | E145K | QGIEFWSNVCDEKMDLAIEAS EAAE |
| FBXW2 | NM_012164 | Missense mutation | G1274A | G425E | SSFLAGEASWLNELDGHNDT GLVFA |
| ZNF592 | NM_014630 | Missense mutation | A1240T | I414F | SKSPVGSPLGSAFAEAPSEMP GDEV |
| NPRL2 | NM_006545 | Missense mutation | G256T | G86C | KKYSRNALLFNLCFVCDAQA KTCAL |
| SMARCA4 | NM _001128845 | Missense mutation | C2729T | T910M | NTHYVAPRRLLLMGTPLQNK LPELW |
| EP300 | NM 001429 | Deletion frame shift | 433delC | Q145fs | SPNMGMGTSGPNRVLRSQQV |
| MGA | NM_001080541 | Deletion frame shift | 4816delC | H1606fs | HPNGQIVQLLPLISFEALIPSPT YSLSCFGTQGL |
| BACE2 | NM_012105 | Missense mutation | C899T | A300V | LNLDCREYNADKVIVDSGTTL LRLP |
| WSB2 | NM_001278557 | Missense mutation | T174A | F58L | TWSVAFSPDGSWLAWSQGHC IVKLI |
| NCBP1 | NM_002486 | Missense mutation | C1070T | A357V | IPLNYHIVEVIFVELFQLPAPPH ID |
| RALGAPB | NM _001282917 | Deletion frame shift | 2763_2766d el | S921fs | LLGAFPSPSGPASVVL |
| EPB41 | NM 203343 - | Missense mutation | C1460T | A487V | SGRTQAQTRQASVLIDRPAPH FERT |
| PNPLA7 | NM_152286 | Missense mutation | C3917G | P1306R | SSLRHRHPSLAFRKLSEGSSDQ DG |
| C3orf58 | NM_001134470 | Missense mutation | G163T | A55S | YFNAPWEKRVDLSWQLMEIA EQLTN |
| TMEM130 | NM_001134451 | Missense mutation | A118G | N40D | AEFLVGDLVVTQDTSLPWPSS YLTK |
| GPR137 | NM_001170880 | Missense mutation | C728G | A243G | SRACYNLTALALGPQSRLDTF DYDW |
| CASS4 | NM_001164115 | Missense mutation | G976A | A326T | TKNAVLTYPSPATLGHLQAEA EKLE |
| MAP2K3 | NM_002756 | Missense mutation | T361C | F121L | ICMELMDTSLDKLYRKVLDK NMTIP |
| RASGRP3 | NM_015376 | Missense mutation | T1376C | V459A | IAANFPFLDSFCALDKDQDGLI SKD |
| DGKD | NM_003648 | Missense mutation | G1901T | R634L | SESFGVPKGRSQLKVSKSPCE KLIS |
| KMT2C | NM_170606 | Missense mutation | G5053T | A1685S | TRVKQIAKLWRKSSSQERAPY VQKA |
| ASRGL1 | NM_001083926 | Missense mutation | C827G | A276G | GLIVVSKTGDWVGKWTSTSM PWAAA |
| CDR2 | NM_001802 | Missense mutation | C242A | A81E | QVELLRQMNEQHEKVYEQLD VTARE |
| NOM1 | NM_138400 | Missense mutation | C1859G | P620R | RWWIVGSAWSGARMIDNSHH THLQK |
| GATA2 | NM_001145662 | Missense mutation | T862A | S288T | SSFTPKQRSKARTCSEGRECV NCGA |
| CRACR2B | NM_001286606 | Missense mutation | A145G | I49V | LFLLCDKEAKGFVTKHDLQG LQSDL |
| COL6A3 | NM_057166 | Missense mutation | G3850A | A1284T | GGRSPTVRVSVVTNTPSGPVE AFDF |
| TMCC1 | NM_001017395 | Missense mutation | C1739T | A580V | QQQVVQLEGLENVTARNLLG KLINI |
| RSBN1 | NM_018364 | Insertion frame shift | 185dupT | V62fs | AAQVGAVRVVRAVGGAGGA GQRGEGETSCWGLPAGS |
| OLFML2B | NM_001297713 | Missense mutation | C13T | R5W | MAKPWLLVLYFALIWP |
| SLC38A7 | NM_001308384 | Missense mutation | G38T | W13L | MAQVSINNDYSELDLSTDAGE RARL |
| B3GALT6 | NM_080605 | Missense mutation | C560T | S187L | PARRRRLYWGFFLGRGRVKP GGRWR |
| MYRIP | NM_001284426 | Missense mutation | G580A | E194K | LAKPKSGTFQALKVASSVASA YDEM |
| PGBD5 | NM_001258311 | Missense mutation | A1148G | N383S | SDCTGLPLSMLTSPATPPARG QYQI |
| DIS3L2 | NM_152383 | Missense mutation | C2183T | A728V | AAALGYRERLDMVPDTLQKQ ADHCN |
| WNK4 | NM_032387 | Missense mutation | G3232A | E1078K | TREALAESDRAAKGLGAGVE EEGDD |
| WBP4 | NM_007187 | Missense mutation | A745C | I249L | EGGVSTETEKPKLKFKEKNKN SDGG |
| CTU2 | NM_001012759 | Missense mutation | C443T | A148V | ILQATGFPWHVVVLEEVFSLP PSVL |
| KMT5A | NM_020382 | Missense mutation | G496C | A166P | KQALKKPIKGKQPPRKKAQG KTQQN |
| DUSP8 | NM_004420 | Missense mutation | G1855C | V619L | AALGKQASFSGSLEVIEVS |
| PCDHA2 | NM_018905 | Missense mutation | G194T | R65L | GLELEELVPRLFLVASKRHGD LLEV |
| CCDC142 | NM_032779 | Missense mutation | C32T | P11L | MAQASRSGSLLPLVIVPPLRA QP |
| CNTNAP3 | NM_033655 | Missense mutation | A3367G | T1123A | EEAVVMVEVNQSAKKQVILS SGTEF |
| TREML1 | NM_001271807 | Missense mutation | T134C | V45A | SILVQCHYRLQDAKAQKVWC RFLPE |
| TMEM191C | NM_001207052 | Missense mutation | T494C | L165P | ELSSQLFYYGGEPQSQKSTEQ QLAA |
| USH2A | NM 206933 - | Deletion frame shift | 14970_1497 1del | T4990fs | DTTLYIPRTADKTLFPGHLHD |
| BARHL2 | NM_020063 | Missense mutation | G836T | W279L | AALNLTDTQVKTLYQNRRTK WKRQT |
| CFHR1 | NM_002113 | Missense mutation | G523C | E175Q | MSKYPSGERVRYQCRSPYEM FGDEE |
| POTEF | NM_001099771 | Missense mutation | T632C | I211T | LNVLDNKKRTALTKAVQCQE DECAL |
| PP2D1 | NM_001252657 | Missense mutation | T896A | L299H | AKAFWRMDRLLGHGRKEVSR VQWSG |
| TOPAZ1 | NM_001145030 | Missense mutation | G3361A | G1121R | RPLCKFAHVPEQRDEKVCMD VFKKY |
| FGF5 | NM_004464 | Missense mutation | G112A | D38N | LAPKGQPGPAATNRNPRGSSS RQSS |
| STPG2 | NM_174952 | Missense mutation | G1293C | E431D | FVKASKRFEESKDITPGPATYE ISQ |

The nucleotide sequences of Tumor-2 tandem antigen genes are as shown in SEQ ID NO. 3 and SEQ ID NO. 4.

**Table 3: Tumor mutations of Tumor-3 and tandem antigen gene sequences**

| **Gene name** | **Reference sequence** | **Mutation type** | **Mutant base** | **Mutant amino acid** | **Tandem antigen sequence** |
|---|---|---|---|---|---|
| PSMA1 | NM_002786 | Missense mutation | A767G | K256R | AQPAQPADEPAERADEPMEH |
| VCP | NM_007126 | Missense mutation | A1309G | I437V | KKMDLIDLEDETVDAEVMNS LAVTM |
| WNK1 | NM_014823 | Missense mutation | G5025C | E1675D | GITIPGISSDVPDSAHKTTASE AKS |
| PABPC1 | NM_002568 | Missense mutation | T1361C | I454T | ARPHPFQNMPGATRPAAPRPP FSTM |
| HSPG2 | NM_001291860 | Missense mutation | T839A | V280D | VTHAPQPLLPGSDRPLPCGPQ EAAC |
| ATN1 | NM_001007026 | Missense mutation | G2146A | A716T | GLPSLPPPPAAPTSGPPLSATQI KQ |
| TP53 | NM_001126115 | Missense mutation | G128A | R43H | YKQSQHMTEVVRHCPHHERC SDSDG |
| PDE4DIP | NM_001002811 - | Deletion frame shift | 1947delT | V649fs | LEKLRQRIHDKAVLWSGL |
| EPRS | NM_004446 | Missense mutation | C4150G | R1384G | RDMKSCQFVAVRGDTGEKLT VAENE |
| PARP14 | NM_017554 | Missense mutation | G3541A | A1181T | FLLHPSDHENIQTFSDEFARRA NGN |
| METAP1 | NM_015143 | Missense mutation | G961A | G321S | YAKNKAVGVMKSSHVFTIEP MICEG |
| IGSF3 | NM_001542 | Missense mutation | G921T | Q307H | GEPVEFRCILEAHNVPDRYFA VSWA |
| LTBP1 | NM_001166264 | Missense mutation | C3814A | Q1272K | LNGCENGRCVRVKEGYTCDC FDGYH |
| TPR | NM_003292 | Missense mutation | A3830G | K1277R | KTETMNVVMETNRMLREEKE RLEQD |
| KCTD3 | NM_016121 | Missense mutation | G763C | E255Q | HGDKDKMVAVASQSSIILWS VQDGG |
| LMBR1L | NM_001300751 | Missense mutation | T565G | F189V | DKNKANRESLYDVWEYYLPY LYSCI |
| TSPAN15 | NM_012339 | Missense mutation | C755T | T252M | GILLPQFLGVLLMLLYITRVED IIM |
| SEMA4A | NM_001193302 | Missense mutation | G233T | R78L | MNNFLGSEPILMLTLGSQPVL KTDN |
| DNM1L | NM_001278466 | Missense mutation | C616G | P206A | RNATGPRPALFVAEVSFELLV KRQI |
| RUBCN | NM_001145642 | Missense mutation | A146G | D49G | LHVEKFISVHENGQSSADGAS ERAV |
| FLT4 | NM_002020 | Missense mutation | G3550T | V1184F | LLQGRGLQEEEEFCMAPRSSQ SSEE |
| PPP4R3A | NM_001284281 | Missense mutation | G421T | A141S | VILGMDDTQVRSSATDIFSYL VEYN |
| MCRS1 | NM_001278341 | Missense mutation | G331C | E111Q | DSKLKDMRDEVLQHELMVA DRRQKR |
| TMEM94 | NM_014738 | Missense mutation | G2380A | A794T | CELPSTIPIKQNTRRSSWSSDE GIG |
| WDFY3 | NM_014991 | Missense mutation | G5543A | R1848H | CTEAVFLLLGMLHSMLTSPW QSEEE |
| PROSER3 | NM_001039887 | Deletion frame shift | 653delC | S218fs | LKRSKASISSSSSSAPAMPALP HSPPALMASLPSRRPSSLTPAR ALAPGHPHPRHQPRPRPPPLL QPQPPPKHPFGQRMTFCTSGG SGGSLNRLREARVTELGCRL |
| MBTPS2 | NM_015884 | Missense mutation | A468T | Q156H | QVVVPGINLPVNHLTYFFTAV LISG |
| ZBTB48 | NM_001278647 | Missense mutation | T1397C | V466A | HIKAKHRNERPHACEFCSHAF TQKA |
| STK16 | NM_001008910 | Missense mutation | G139A | A47T | DLVEGLHDGHFYTLKRILCHE QQDR |
| TLR1 | NM_003263 | Missense mutation | A7G | S3G | MTGIFHFAIIFMLIL |
| NOTCH2 | NM_024408 | Missense mutation | C6765A | N2255K | RLHPVPVPADWMKRMEVNE TQYNEM |
| NLRC5 | NM_032206 | Deletion frame shift | 1519_1522 del | Q507fs | TSFCVCTGPGHQQAMLSPTSA CRSFLLPCT |
| ACAP1 | NM_014716 | Missense mutation | G1624A | V542M | RGGRGRPRGQPPMPPKPSIRP RPGS |
| PIK3AP1 | NM_152309 | Missense mutation | G2347T | A783S | MSLERPPRVPPRSASQRPPTRE TFH |
| MICAL3 | NM_015241 | Missense mutation | A4597G | K1533E | PFADDVEDTYDDETEDSSLQE KFFT |
| FYB | NM_001243093 | Insertion frame shift | 1002dupG | P335fs | FPKAPSKLTVGGAMGPKSGK GKGRQEFSHPETEAIASLVYL GSTSTKTQQTTKC |
| PTBP1 | NM_002819 | Missense mutation | C686T | A229V | TKNNQFQALLQYVDPVSAQH AKLSL |
| FILIP1L | NM_001282794 | Missense mutation | C2551G | R851G | RPASPSAPLQDNGTQGLINGA LNKT |
| SLC35A5 | NM_017945 | Missense mutation | G1199A | R400H | ERIRDLSGNLWEHSSGDGEEL ERLT |
| CRYBG3 | NM_153605 | Missense mutation | T3243G | N1081K | EVSMIVNSHKPQKNLDSIQVT KDLT |
| USP1 | NM_001017415 | Missense mutation | C107G | A36G | SLKFFQKKETKRGLDFTDSQE NEEK |
| MRPS11 | NM_176805 | Missense mutation | A265G | K89E | ASCGTEGFRNAKEGTGIAAQT AGIA |
| LDLRAD4 | NM_001276251 | Missense mutation | G88T | A30S | LWPSDSAAPRLGSSEIMHAPR SRDR |
| LDB2 | NM_001130834 | Missense mutation | T200C | L67P | ATEFFEDDATLTPSFCLEDGP KRYT |
| CEP192 | NM_032142 | Missense mutation | C1082A | T361N | NSECASKDVLVKNLRAIDVKL NSDN |
| PTK2B | NM_173175 | Missense mutation | A2108G | K703R | MEQERNARYRTPRILEPTAFQ EPPP |
| CCDC191 | NM_020817 | Missense mutation | G1462A | G488S | PPLWEKPPLGSSSCMLSPPLG RTTT |
| SHPRH | NM_001042683 | Missense mutation | C3890G | T1297R | PTTTRGLWAISERERSMKAILS FAK |
| TMEM2 | NM_001135820 | Missense mutation | T3731C | L1244P | IKQLNISHLLVPPGLAKPAHLY DKG |
| ANKRD20A4 | NM_001098805 | Missense mutation | A2222T | N741I | CVEERICHLQREIAWLVQQLD DVHQ |
| ANAPC1 | NM_022662 | Missense mutation | G1438C | A480P | FGSVTNIPAKDAPPVEKIDTM LVLE |
| SLC16A1 | NM_001166496 | Missense mutation | G402A | M134I | LGLAFNLNPALTIIGKYFYKR RPLA |
| AOC3 | NM_001277731 | Missense mutation | G142A | A48T | SQLPHCPSVSPSTQPWTHPGQ SQLF |
| SLIT2 | NM_001289135 | Missense mutation | G2150A | C717Y | DFTCDDGNDDNSYSPLSRCPT ECTC |
| KDELC1 | NM_024089 | Missense mutation | A1058G | H353R | KHDENLYGPIVKRISFFDFFKH KYQ |
| PRKRIR | NM_004705 | Missense mutation | T1828G | S610A | HLKALKCLSLVPAVMGQLKF NTSEE |
| TLE1 | NM_001303103 | Missense mutation | T2255A | V752E | GASIFQSKESSSELSCDISVDD KYI |
| TET2 | NM_001127208 | Missense mutation | A4742T | N1581I | YMRRPNPVSPYPISSHTSDIYG STS |
| CITED2 | NM_001168388 | Missense mutation | C406G | P136A | FNHHPYPHNHYMADLHPAAG HQMNG |
| WNT3A | NM_033131 | Missense mutation | G488A | G163E | KWGGCSEDIEFGEMVSREFA DAREN |

The nucleotide sequences of Tumor-3 tandem antigen genes are as shown in SEQ ID NO. 5 and SEQ ID NO. 6.

**Table 4: Information of amino acid sequences of TCR a and b chains**

| **Tumor-1** | **a-V gene** | **a-J gene** | **a-CDR3** | **b-V gene** | **b-D gene** | **b-J gene** | **b-CDR3** |
|---|---|---|---|---|---|---|---|
| CD4 TCR-1 | TRAV29DV5 | TRAJ54 | CAASPIIQGAQKL | TRBV19 | TRBD1 | TRBJ2-7 | CASKISGTGLYEQ YF |
| CD4 TCR-2 | TRAV3 | TRAJ40 | CAVRDRAYKYIF | TRBV9 | TRBD2 | TRBJ2-1 | CASSPRGTSENEQ FF |
| CD4 TCR-3 | TRAV21 | TRAJ37 | CALPGNTGKLIF | TRBV4-1 | TRBD2 | TRBJ2-1 | CASSQVTSGRGL YNEQFF |
| CD4 TCR-4 | TRAV22 | TRAJ49 | CAVFTGNQFYF | TRBV4-3 | TRBD1 | TRBJ1-2 | CASSQIGGYGYTF |
| CD4 TCR-5 | TRAV12-1 | TRAJ6 | CASGSGGSYIPTF | TRBV15 | TRBD2 | TRBJ2-1 | CATSRTSGSYEQF F |
| CD4 TCR-6 | TRAV8-2 | TRAJ49 | CVVSPNTGNQFY F | TRBV7-3 | None | TRBJ1-2 | CASSLFGEGYTF |
| CD4 TCR-7 | TRAV27 | TRAJ57 | CAGVREGGSEKL VF | TRBV5-1 | None | TRBJ2-1 | CASSKVVNSYNE QFF |
| CD4 TCR-8 | TRAV8-2 | TRAJ49 | CVVSPNTGNQFY F | TRBV11-2 | TRBD2 | TRBJ2-7 | CASGEGSGVSYE QYF |
| CD4 TCR-9 | TRAV8-6 | TRAJ53 | CAVSTNSGGSNY KLTF | TRBV6-5 | TRBD1 | TRBJ2-3 | CASSLNRGFSDTQ YF |
| CD4 TCR-10 | TRAV13-1 | TRAJ44 | CAAREYGTASKL TF | TRBV5-8 | TRBD2 | TRBJ1-5 | CASSPGTGNQPQ HF |

| **Tumor-1** | **a-V gene** | **a-J gene** | **a-CDR3** | **b-V gene** | **b-D gene** | **b-J gene** | **b-CDR3** |
|---|---|---|---|---|---|---|---|
| CD8 TCR-1 | TRAV13-1 | TRAJ10 | CAATLWGGGNK LTF | TRBV20-1 | TRBD1 | TRBJ1-1 | CSATNGGTEAFF |
| CD8 TCR-2 | TRAV3 | TRAJ40 | CAVRDSFTSGTY KYIF | TRBV27 | TRBD1 | TRBJ2-7 | CASSWTGAPYEQ YF |
| CD8 TCR-3 | TRAV35 | TRAJ34 | CAGQSYTDKLIF | TRBV29-1 | TRBD1 | TRBJ2-1 | CSVEGTGEYNEQ FF |
| CD8 TCR-4 | TRAV3 | TRAJ44 | CAVRPSTGTASK LTF | TRBV5-1 | TRBD2 | TRBJ2-3 | CASSPGTSGRPFP TDTQYF |
| CD8 TCR-5 | TRAV25 | TRAJ53 | CAGLSGGSNYKL TF | TRBV10-3 | TRBD1 | TRBJ1-4 | CAIRTESEKLFF |
| CD8 TCR-6 | TRAV19 | TRAJ22 | CALSEATGSARQ LTF | TRBV27 | TRBD2 | TRBJ2-1 | CASSWFSANDEQ FF |
| CD8 TCR-7 | TRAV29DV5 | TRAJ58 | CAASGTSGSRLTF | TRBV20-1 | TRBD1 | TRBJ1-2 | CSANRGNYGYFF |
| CD8 TCR-8 | TRAV12-2 | TRAJ15 | CAVNIQAGTALIF | TRBV19 | TRBD2 | TRBJ2-5 | CASSDRSPGSGLE TQYF |
| CD8 TCR-9 | TRAV12-2 | TRAJ22 | CAVISSGSARQLT F | TRBV7-2 | None | TRBJ2-1 | CASSLVRNEQFF |
| CD8 TCR-10 | TRAV3 | TRAJ45 | CAVRAPGGGADG LTF | TRBV5-8 | TRBD2 | TRBJ2-1 | CASSFWREHNEQ FF |

| **Tumor-2** | **a-V gene** | **a-J gene** | **a-CDR3** | **b-V gene** | **b-D gene** | **b-J gene** | **b-CDR3** |
|---|---|---|---|---|---|---|---|
| CD4 TCR-1 | TRAV29DV5 | TRAJ42 | CAASANYGGSQG NLIF | TRBV20-1 | TRBD2 | TRBJ2-7 | CSARAGRPPNAQ YF |
| CD4 TCR-2 | TRAV12-3 | TRAJ4 | CAMFGGYNKLIF | TRBV7-3 | None | TRBJ2-2 | CASSLNGQNTGE LFF |
| CD4 TCR-3 | TRAV9-2 | TRAJ12 | CALSSGSSYKLIF | TRBV12-3 | None | TRBJ1-6 | CASSLDGNSPLHF |
| CD4 TCR-4 | TRAV8-4 | TRAJ13 | CAVSDSGSGGYQ KVTF | TRBV24-1 | TRBD2 | TRBJ2-2 | CATSDSGLAWNT GELFF |
| CD4 TCR-5 | TRAV5 | TRAJ26 | CAEESGQNFVF | TRBV9 | TRBD2 | TRBJ2-3 | CASSVGRVSTDT QYF |
| CD4 TCR-6 | TRAV21 | TRAJ37 | CAVGSGNTGKLIF | TRBV6-5 | TRBD2 | TRBJ2-1 | CASTRTSGRPNNE QFF |
| CD4 TCR-7 | TRAV4 | TRAJ20 | CLVDNDYKLSF | TRBV30 | TRBD1 | TRBJ2-7 | CAWSVQGDRAY YEQYF |
| CD4 TCR-8 | TRAV21 | TRAJ44 | CAVCAGTASKLT F | TRBV12-3 | TRBD2 | TRBJ2-7 | CASSPRPAGDNE QYF |
| CD4 TCR-9 | TRAV12-3 | TRAJ10 | CAMTLTGGGNKL TF | TRBV4-2 | TRBD1 | TRBJ2-4 | CASSQEDRSGNIQ YF |
| CD4 TCR-10 | TRAV17 | TRAJ52 | CATELVVGTSYG KLTF | TRBV12-5 | TRBD2 | TRBJ1-4 | CASGLVPGEKLFF |

| **Tumor-2** | **a-V gene** | **a-J gene** | **a-CDR3** | **b-V gene** | **b-D gene** | **b-J gene** | **b-CDR3** |
|---|---|---|---|---|---|---|---|
| CD8 TCR-1 | TRAV17 | TRAJ9 | CATPDRGGFKTIF | TRBV5-1 | TRBD1 | TRBJ1-2 | CASSLAGDMTGY TF |
| CD8 TCR-2 | TRAV25 | TRAJ52 | CAGPSNAGGTSY GKLTF | TRBV6-5 | TRBD1 | TRBJ1-2 | CASSPRTHGSYTF |
| CD8 TCR-3 | TRAV38-2DV8 | TRAJ54 | CAYRSWVRGIQG AQKLVF | TRBV5-1 | TRBD1 | TRBJ1-1 | CASSPGQGARTE AFF |
| CD8 TCR-4 | TRAV38-2DV8 | TRAJ30 | CAYLVDKIIF | TRBV5-1 | TRBD1 | TRBJ2-7 | CASSPTGGPYEQ YF |
| CD8 TCR-5 | TRAV20 | TRAJ10 | CAVILTGGGNKL TF | TRBV18 | TRBD1 | TRBJ2-3 | CASSDSGTDTQY F |
| CD8 TCR-6 | TRAV12-1 | TRAJ52 | CGTIVGGTSYGK LTF | TRBV6-3 | TRBD2 | TRBJ1-2 | CASSFLDWEANY GYTF |
| CD8 TCR-7 | TRAV5 | TRAJ27 | CAERDTNAGKST F | TRBV7-8 | None | TRBJ1-1 | CASSLAPGDTEAF F |
| CD8 TCR-8 | TRAV5 | TRAJ4 | CAEALSGGYNKL IF | TRBV7-9 | TRBD1 | TRBJ1-1 | CASSFTGETEAFF |
| CD8 TCR-9 | TRAV38-2DV8 | TRAJ41 | CAPSNSGYALNF | TRBV12-4 | TRBD2 | TRBJ2-2 | CASSLPGRRNTGE LFF |
| CD8 TCR-10 | TRAV12-1 | TRAJ29 | CVLDSGNTPLVF | TRBV14 | None | TRBJ2-7 | CASSPVGPSYEQY F |

| Tumor-3 | **a-V** gene | **a-J** gene | **a-CDR3** | b-V gene | b-D gene | b-J gene | **b-CDR3** |
|---|---|---|---|---|---|---|---|
| CD4 TCR-1 | TRAV8-4 | TRAJ20 | CAVSDRANDYKL SF | TRBV6-5 | TRBD1 | TRBJ1-5 | CASRPFRDSNQPQ HF |
| CD4 TCR-2 | TRAV5 | TRAJ20 | CAAAATNDYKLS F | TRBV5-4 | TRBD1 | TRBJ1-2 | CASRTTGDYGYT F |
| CD4 TCR-3 | TRAV4 | TRAJ10 | CLVGDPPLGGGN KLTF | TRBV7-3 | TRBD1 | TRBJ1-1 | CASSFSRGGEAFF |
| CD4 TCR-4 | TRAV5 | TRAJ23 | CAEGVYNQGGKL IF | TRBV7-8 | TRBD1 | TRBJ2-7 | CASSLAVGGEQY F |
| CD4 TCR-5 | TRAV14DV4 | TRAJ15 | CAMRPHQAGTAL IF | TRBV6-1 | TRBD2 | TRBJ1-2 | CAVGGGPGTNYG YTF |
| CD4 TCR-6 | TRAV9-2 | TRAJ49 | CALSGWGSGNQF YF | TRBV7-3 | TRBD1 | TRBJ2-2 | CASSLTGTDNTG ELFF |
| CD4 TCR-7 | TRAV12-3 | TRAJ30 | CAMSAPYRDDKII F | TRBV7-9 | TRBD1 | TRBJ2-7 | CASSSDMTESYE QYF |
| CD4 TCR-8 | TRAV13-2 | TRAJ8 | CAEKKGFQKLVF | TRBV4-1 | TRBD2 | TRBJ1-1 | CASSPGEGGGTE AFF |
| CD4 TCR-9 | TRAV29DV5 | TRAJ13 | CAASKVTF | TRBV11-2 | TRBD1 | TRBJ1-6 | CASTPGTGYSPLH F |
| CD4 TCR-10 | TRAV38-2DV8 | TRAJ42 | CAYRSPNYGGSQ GNLIF | TRBV7-2 | TRBD1 | TRBJ2-7 | CASSLGRGGIYEQ YF |

| Tumor-3 | **a-V** gene | **a-J** gene | **a-CDR3** | b-V gene | b-D gene | b-J gene | **b-CDR3** |
|---|---|---|---|---|---|---|---|
| CD8 TCR-1 | TRAV13-1 | TRAJ20 | CAAGDDYKLSF | TRBV20-1 | None | TRBJ1-1 | CSARDPRRTNTE AFF |
| CD8 TCR-2 | TRAV25 | TRAJ38 | CAGASNAGNNRK LIW | TRBV5-4 | TRBD2 | TRBJ2-3 | CASSLLLARRSDT QYF |
| CD8 TCR-3 | TRAV5 | TRAJ17 | CVLPPAAGNKLT F | TRBV2 | TRBD1 | TRBJ1-3 | CASSEQGSGNTIY F |
| CD8 TCR-4 | TRAV41 | TRAJ42 | CAVRRDGGSQGN LIF | TRBV6-1 | TRBD2 | TRBJ1-4 | CASTPRGKGEKL FF |
| CD8 TCR-5 | TRAV19 | TRAJ32 | CALGRRGGATNK LIF | TRBV5-1 | TRBD2 | TRBJ2-5 | CASSTGLAGQET QYF |
| CD8 TCR-6 | TRAV36DV7 | TRAJ53 | CAVESGGSNYKL TF | TRBV18 | TRBD1 | TRBJ1-1 | CASSLDWENTEA FF |
| CD8 TCR-7 | TRAV38-2DV8 | TRAJ41 | CARYSGYALNF | TRBV18 | TRBD2 | TRBJ2-1 | CASSPELADYNE QFF |
| CD8 TCR-8 | TRAV12-2 | TRAJ52 | CAVKSHRGTSYG KLTF | TRBV7-3 | None | TRBJ2-2 | CASSFGPGELFF |
| CD8 TCR-9 | TRAV13-1 | TRAJ23 | CAASPRIYNQGG KLIF | TRBV19 | TRBD1 | TRBJ2-2 | CASSAVDRPTGE LFF |
| CD8 TCR-10 | TRAV20 | TRAJ33 | CAVRGMDSNYQ LIW | TRBV7-2 | TRBD1 | TRBJ2-7 | CASSLVGGSYEQ YF |

In order to verify whether these TCRs recognize tumor antigens, the B cells expressing the tumor antigens were mixed with each TCR-T cell line and cultured for antigen stimulation experiment, and the B cells not expressing tumor antigens were used as a control.

Firstly, the above B cells were mixed with the T cells at a ratio of 1:1, inoculated in a 96-well plate, and co-cultured for 48 h, and then, the IFNG level in the supernatant was detected by ELISA. If the tumor antigens expressed by the B cells could be recognized by the TCRs expressed by the T cells, then the T cells would be activated and secrete the effector cytokine IFNG. The more the T cells being activated, the more the generated IFNG. By comparing the level of IFNG in the supernatant, whether these TCR-T cells could be activated by the tumor antigens can be verified, so as to determine whether these TCRs can recognize the tumor antigens.

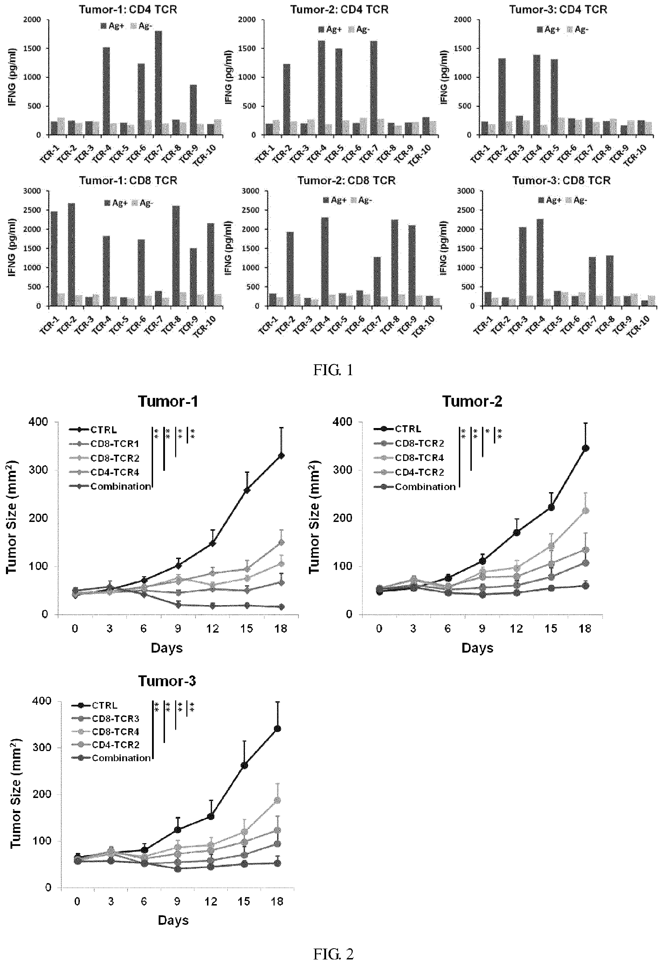

As shown in Fig. 1, the TCRs that recognized tumor antigens can be identified accurately using the established approach. Among the high-frequency TCRs of the first tumor tissue, four TCRs derived from CD4 T cell and seven TCRs derived from CD8 T cell can be activated by the B cells expressing the tumor antigens. The TCRs identified in the second tumor tissue that can recognize tumor antigens include 4 TCRs from CD4 T cells and 5 TCRs from CD8 T cells, respectively. In the third tumor tissue, there are 3 TCRs from CD4 T cells and 4 TCRs from CD8 T cells. These results indicate that both T cells that can recognize tumor antigens and T cells that do not recognize tumor antigens are indeed present in the tumor.

Next, in order to verify whether these tumor-specific TCRs can be used for tumor therapy, the TCR-T cells that recognize tumor antigens were used to treat a tumor PDX model of the same origin. From the TCRs that have been verified to recognize tumor antigens, two TCRs with the highest frequency in CD8 T cells and one TCR with the highest frequency in CD4 T cells were selected and prepared for TCR-T cells for treatment. In addition, the three TCR-T cells were also mixed at a ratio of 1:1:1 for multi-TCR treatment. When the PDX tumors transplanted subcutaneously into NSG mice grew to about 50 mm² in size, 6 × 10⁶ TCR-T cells were injected into the mice through the tail vein.

As shown in Fig. 2, in the treatments for the three tumor PDXs, the treatment with the mixed three TCRs shows the best therapeutic effect, and the treatments with the single TCR also all show obvious therapeutic effects.

These results show that the tumor antigen-recognizing TCRs identified by the method according to the present disclosure not only recognize the tumor antigens, but also effectively treat the tumor, and the treatments with multiple TCRs simultaneously produces better therapeutic effect.

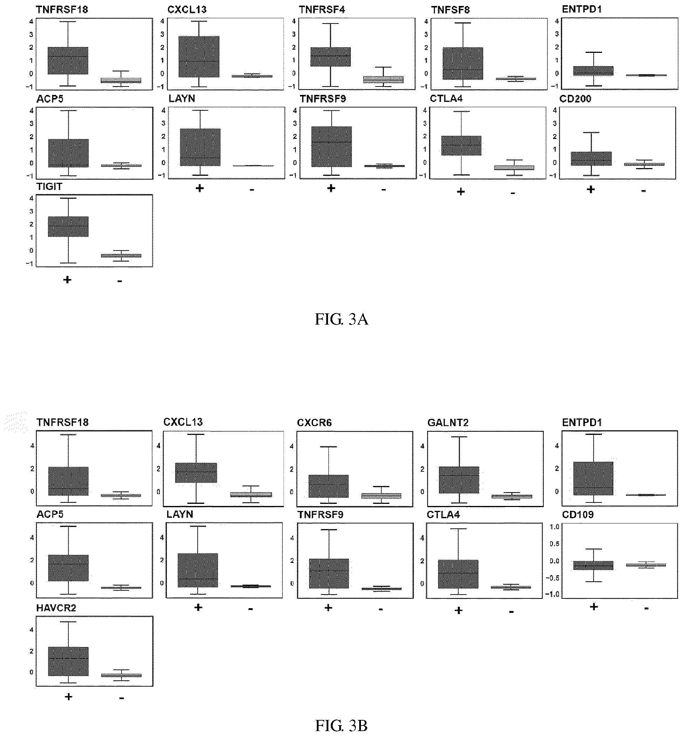

In order to identify the molecular markers of the subset of tumor antigen-specific T cells, CD4 and CD8 T cells carrying these TCRs were divided into two groups: tumor antigen-recognizing positive group and tumor antigen recognizing negative group, depending on whether the identified TCRs recognize tumors. Then, using the RNA sequencing results of each cell, the abundance of RNA expression of each gene was compared between the two groups of T cells to find out the differentially expressed genes. As a result, a number of genes specifically expressed in the T cells positive in tumor antigen recognition were found out. As shown, the tumor antigen-recognizing CD4 T cells specifically express: TNFRSF18, CXCL13, TNFRSF4, TNFSF8, ENTPD1, ACP5, LAYN, TNFRSF9, CTLA4, CD200 and TIGIT (Fig. 3, (a)), and the tumor antigen-recognizing CD8 T cells specifically express: TNFRSF18, CXCL13, CXCR6, GALNT2, ENTPD1, ACP5, HAVCR2, LAYN, TNFRSF9, CTLA4 and CD109 (Fig. 3, (b)). Among them, TNFRSF18, CXCL13, ENTPD1, ACP5, LAYN, TNFRSF9 and CTLA4 are highly expressed in both the tumor antigen-recognizing CD4 and CD8 T cells. By these specifically expressed genes, the T cells that recognize tumor antigens can be significantly distinguished from the T cells that do not recognize the tumor antigens. In addition, these specifically expressed genes can all be used to significantly distinguish the T cells that recognize tumor antigens from the T cells that do not recognize the tumor antigens in various tumor types, thus having a good broad spectrum. Therefore, these genes can be used as molecular markers to identify and isolate T cells that recognize tumor antigens from tumors and obtain the TCRs carried thereby for tumor treatment.

In order to further verify whether the T cells that recognize tumor antigens can be identified and isolated by these molecular markers, the molecular markers expressed on the surface of the T cells were selected as molecular markers for flow sorting, wherein TNFRSF18, TNFRSF4, ENTPD1, TNFRSF9, CTLA4, CD200 and TIGIT were selected for the tumor antigen-recognizing CD4 T cell, and TNFRSF18, CXCR6, ENTPD1, TNFRSF9, HAVCR2, CTLA4 and CD109 were selected for the tumor antigen-recognizing CD8 T cell. Firstly, according to the above-mentioned tumor tissue digestion method, five lung cancer tissues were digested into single-cell suspensions, and a portion of the single-cell suspension was centrifuged, resuspended in RPMI medium containing 10% FBS at a density of 1 × 10⁶ cells/ml, and then frozen to -80°C, as tumor antigens for the subsequent antigen stimulation experiment. The remaining single-cell suspension was divided into seven aliquots, which were respectively subjected to flow antibody stain. The antibody combinations were 1) CD4, CD8 and TNFRSF18; 2) CD4, CD8 and ENTPD1; 3) CD4, CD8 and TNFRSF9; 4) CD4, CD8 and CTLA4; 5) CD4, CD8, TNFRSF4 and CXCR6; 6) CD4, CD8, CD200 and HAVCR2; and 7) CD4, CD8, TIGIT and CD109. Then, the CD4 and CD8 T cells in the tumor tissue were sorted into two groups of molecular marker positive and negative cells respectively according to the staining of these molecular marker antibodies, and 14 groups of CD4 T cells and 14 groups of CD8 T cells were totally obtained.

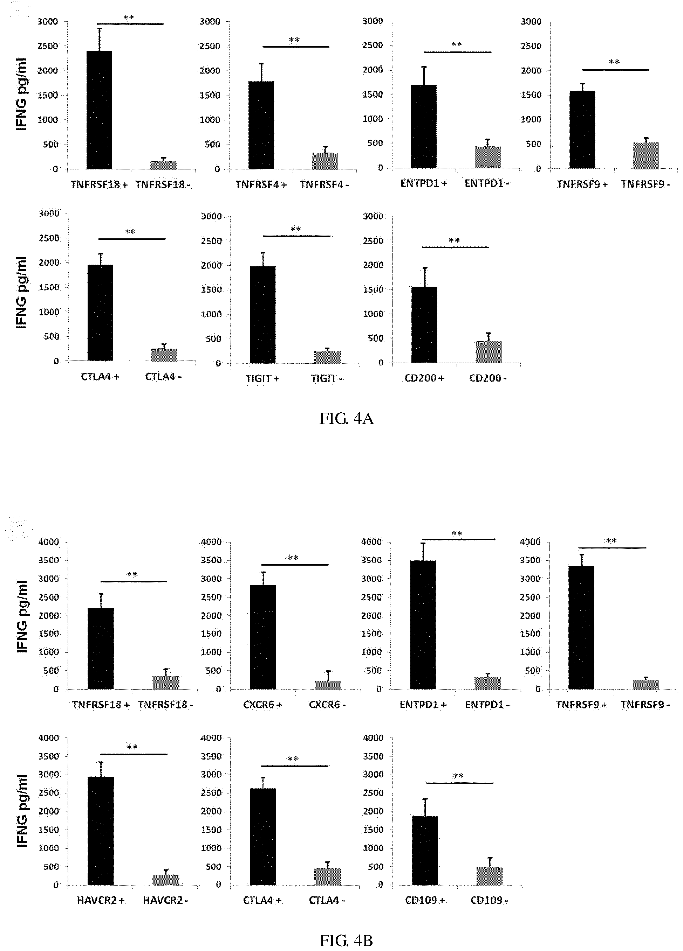

Each sorted T cells group was resuspended in RPMI medium containing 10% FBS and 200 ng/ml IL2 and inoculated into a U-shaped bottom 96-well plate coated with CD3/CD28 antibody for nonspecific activation. After one week of incubation, the T cells of each group were taken out. After washing with PBS twice, the T cells were resuspended in RPMI medium containing 10% FBS at a density of 2 × 10⁶/ml. 200 µL of cells were inoculated into a U-shaped bottom 96-well plate, and each group of cells were set in triplicate. In addition, the frozen single-cell suspension of tumor tissue was revived to 37°C by a water bath, from which 50 µL was taken and added to the inoculated T cells as tumor antigen for specific activation. After activation for 20 hours, the content of IFNG in the culture medium was detected by ELISA to measure the response of the T cells in each group to the tumor antigen stimulation. The inventors found that among CD4 T cells, TNFRSF18+, TNFRSF4+, ENTPD1+, TNFRSF9+, CTLA4+, CD200+ and TIGIT+ T cells could all be activated by the tumor antigens, whereas the corresponding T cells negative in these molecular markers could not be activated (Fig. 4, (a)). Similarly, among CD8 T cells, TNFRSF18+, CXCR6+, ENTPD1+, TNFRSF9+, HAVCR2+, CTLA4+ and CD109+ T cells could all be activated by the tumor antigens, whereas the corresponding T cells negative in these molecular markers could not be activated (Fig. 4, (b)).

The above results indicate that these molecular markers can be used to isolate tumor antigen-recognizing T cells from tumors. The T cells separated and obtained by these molecular markers and the TCRs carried thereby can be used for the treatment of a tumor in a patient.

## Claims

1. A TCR-T cell for tumor-killing, wherein the TCR-T cell is a T cell carrying a TCR that recognizes a tumor antigen, and the TCR in the TCR-T cell is derived from any one or more of the following T cells:
1) a CD4 T cell expressing one or more of TNFRSF18, CXCL13, TNFRSF4, TNFSF8, ENTPD1, ACP5, LAYN, TNFRSF9, CTLA4, CD200 and TIGIT genes in the tumor; and
2) a CD8 T cell expressing one or more of TNFRSF18, CXCL13, CXCR6, GALNT2, ENTPD1, ACP5, HAVCR2, LAYN, TNFRSF9, CTLA4 and CD109 genes in the tumor.

2. Use of the TCR-T cell according to claim 1 in the preparation of a medicine for treating a tumor.

3. The use according to claim 2, wherein the tumor is any one or more selected from the group consisting of lung cancer, melanoma, intestinal cancer, liver cancer, stomach cancer, breast cancer, cervical cancer, ovarian cancer, kidney cancer, bladder cancer and esophageal cancer.

4. A method for preparing the TCR-T cell according to claim 1, comprising: introducing one or more nucleotide sequences of a TCR that recognizes a tumor antigen into a T cell for expression to construct the TCR-T cell.

5. The method according to claim 4, wherein the step of identifying the TCR that recognizes the tumor antigen comprises: by means of flow sorting, magnetic bead separation or tumor tissue in-situ sequencing, obtaining, from a tumor tissue, a T cell, which expresses any one or more of the markers of a CD4 T cell for tumor antigen recognition, selected from the group consisting of TNFRSF18, CXCL13, TNFRSF4, TNFSF8, ENTPD1, ACP5, LAYN, TNFRSF9, CTLA4, CD200 and TIGIT, and a TCR sequence carried thereby; and a T cell, which expresses any one or more of the markers of a CD8 T cell for tumor antigen recognition, selected from the group consisting of TNFRSF18, CXCL13, CXCR6, GALNT2, ENTPD1, ACP5, HAVCR2, LAYN, TNFRSF9, CTLA4 and CD109, and a TCR sequence carried thereby.

6. A method for identifying a T cell and a TCR for tumor antigen recognition, comprising: establishing a TCR-expressing TCR-T cell by cloning a high-frequency TCR in a tumor tissue, performing *in vitro* tumor antigen stimulation by using antigen-presenting cells expressing a tumor antigen tandem gene, and identifying a TCR carried by a TCR-T cell that can be stimulated as the TCR for tumor antigen recognition, and the T cell carrying such TCR as the T cell for tumor antigen recognition,
wherein the TCR in the TCR-T cell is derived from any one or more of the following T cells:
1) a CD4 T cell expressing one or more of TNFRSF18, CXCL13, TNFRSF4, TNFSF8, ENTPD1, ACP5, LAYN, TNFRSF9, CTLA4, CD200 and TIGIT genes in the tumor; and
2) a CD8 T cell expressing one or more of TNFRSF18, CXCL13, CXCR6, GALNT2, ENTPD1, ACP5, HAVCR2, LAYN, TNFRSF9, CTLA4 and CD109 genes in the tumor.
